Europäisches Patentamt

European Patent Office

Office européen des brevets

(19)

(11) Publication number: **0 022 621**
**A1**

(12)

# EUROPEAN PATENT APPLICATION

(21) Application number: 80301964.5

(22) Date of filing: 11.06.80

(51) Int. Cl.³: **C 07 C 103/52, A 61 K 37/02**

(30) Priority: 11.06.79 US 47433

(71) Applicant: **ELI LILLY AND COMPANY, 307, East McCarty Street, Indianapolis Indiana (US)**

(43) Date of publication of application: **21.01.81**
**Bulletin 81/3**

(72) Inventor: **Shields, James Edwin, 7229 Wynter Way, Indianapolis, Indiana (US)**

(84) Designated Contracting States: **DE GB LU NL SE**

(74) Representative: **McVey, Kenneth William Henry et al, Erl Wood Manor, Windlesham Surrey, GU20 6PH (GB)**

(54) **Peptides related to somatostatin, their preparation and pharmaceutical compositions containing them.**

(57) Novel tetradecapeptides of the formula

$$X-Cys-Lys-Y-Phe-Phe-D-Trp$$
$$HO-D-Cys-Ser-Thr-Z-Thr-Lys$$

wherein

X is H-Ala-Gly, H-Ala-D-Ala, H-D-Ala-Gly, H-D-Val-Gly, H-Ala-D-Val, H-Ala-D-Ser, or H-D-Ser-Gly;

Y is Ala or D-Ala; and

Z is Phe, D-Phe, or Cha;

or a non-toxic, pharmaceutically acceptable acid addition salt thereof, which are prepared by reacting the corresponding linear tetradecapeptides with an oxidizing agent, are described herein. These novel peptides inhibit the secretion of growth hormone.

ACTORUM AG

## PEPTIDES RELATED TO SOMATOSTATIN

This invention relates to synthetic peptides structurally related to somatostatin and to intermediates employed in the synthesis thereof.

Somatostatin is the cyclic disulfide tetradecapeptide of the formula:

$$H-Ala-Gly-Cys-Lys-Asn-Phe-Phe-Trp$$
$$HO-Cys-Ser-Thr-Phe-Thr-Lys$$

I

This peptide (I) has been identified as the "somatotropin-release inhibiting factor" (SRIF) which is secreted by the hypothalamus and regulates the secretion of pituitary growth hormone (GH) (somatotropin). [See Brazeau et al., Science, 179, 77 (1973), Burgus et al., Proc. Nat. Acad. Sci. (USA), 70, 684 (1973), and Ling et al., Biochemical and Biophysical Res. Communications, 50, 127 (1973)]. The reduced form of somatostatin is the linear tetradecapeptide of the formula:

$$H-Ala-Gly-Cys-Lys-Asn-Phe-Phe-Trp$$
$$HO-Cys-Ser-Thr-Phe-Thr-Lys$$     II

The reduced form (II) has been prepared by total synthesis, [see Rivier et al., C. R. Acad. Sci. p. Sci. Natur. (Paris), 276, 2737 (1973) and Sarantakis and McKinley, Biochem. and Biophys. Res. Communications, 54, 234 (1973)] and it (II) can be converted to somatostatin (I) by oxidation whereby a bridging bond is formed between the two sulfhydryls of the two cysteinyl amino acid residues in the tetradecapeptide.

Somatostatin inhibits the release of numerous hormones in addition to growth hormone, including those from the pituitary (prolactin), the gut (gastrin, cholecystokinin, and secretin), and the pancreas (insulin and glucagon).

Various polypeptides which may be regarded as structural modifications of somatostatin have been prepared synthetically and are reported in the chemical literature. Such polypeptides have certain structural features in common with somatostatin and differ from somatostatin in that specific amino acid residues or functional groups originally present in the somatostatin molecule are either missing or are replaced by other amino acid residues or functional groups. The present invention relates to novel synthetic biologically active polypeptides which may be regarded as structural modifications of somatostatin. The polypeptides of the invention differ from somatostatin in the following respects:

(a) The H-Ala$^1$-Gly$^2$ segment is either present or is replaced by H-Ala-D-Ala, H-D-Ala-Gly, H-D-Val-Gly, H-Ala-D-Val, H-Ala-D-Ser, or H-D-Ser-Gly;

(b) The Asn$^5$ residue is replaced by Ala or D-Ala;

(c) The Trp$^8$ residue is replaced by D-Trp;

(d) The Phe$^{11}$ residue is either present or is replaced by D-Phe or Cha; and

(e) The Cys$^{14}$ residue is replaced by D-Cys.

X-5201                                    -3-

All optically active amino acids and amino acid residues in the polypeptides depicted and described herein are in the natural or L-configuration, unless otherwise noted.  The symbols identifying the amino acids and the amino acid residues in the polypeptides described herein are those adopted by the IUPAC-IVB Committee on Biochemical Nomenclature Recommendation (1971), and are described in the <u>Archives of Biochemistry and Biophysics</u>, <u>150</u>, 1-8 (1972).

The following abbreviations, most of which are well known and commonly used in the art, are employed herein:

Ala - Alanine

Asn - Asparagine

Cha - Cyclohexylalanine

Cys - Cysteine

Gly - Glycine

Lys - Lysine

Phe - Phenylalanine

Ser - Serine

The - Threonine

Trp - Tryptophan

DCC - N,N'-Dicyclohexylcarbodiimide

BOC - <u>t</u>-Butyloxycarbonyl

PMB - <u>p</u>-Methoxybenzyl

CPOC - Cyclopentyloxycarbonyl

Bzy - Benzyl

BpOC - 2-(<u>p</u>-biphenylyl)isopropyloxycarbonyl

This invention relates to novel tetra-decapeptides of the formula:

$$X-Cys-Lys-Y-Phe-Phe-D-Trp$$
$$|\qquad\qquad\qquad\qquad|$$
$$HO-D-Cys-Ser-Thr-Z-Thr-Lys$$

III

wherein:

X is H-Ala-Gly, H-Ala-D-Ala, H-D-Ala-Gly, H-D-Val-Gly, H-Ala-D-Val, H-Ala-D-Ser, or H-D-Ser-Gly;

Y is Ala or D-Ala; and

Z is Phe, D-Phe or Cha;

or a non-toxic, pharmaceutically acceptable acid addition salt thereof.

The compounds of Formula III are prepared by reacting a corresponding linear compound of the formula:

$$X-Cys-Lys-Y-Phe-Phe-D-Trp-Lys$$
$$|$$
$$HO-D-Cys-Ser-Thr-Z-Thr$$

IV

where the symbols are defined as above, with an oxidizing agent.

The peptides of Formula III are biologically active and inhibit the secretion of growth hormone, as demonstrated *in vivo* in rats using standard pharmacological test procedures. Thus, the peptides are especially useful in the treatment of acromegaly and of other pathological conditions (e.g. diabetes) characterized by the abnormally high secretion of growth

X-5201                                    -5-

hormone. As demonstrated in dogs, certain compounds, moreover, selectively inhibit the secretion of growth hormone i.e. the compounds inhibit growth hormone secretion at a dose which is substantially lower than the dose required to inhibit the secretion of insulin or glucagon.

Preferred embodiments of the peptides defined by Formula III are those wherein X is H-D-Ala-Gly or H-D-Ser-Gly. Especially preferred embodiments are those compounds wherein:

(i)     X is H-D-Ala-Gly; Y is D-Ala, and Z is Phe (i.e. D-Ala$^1$, D-Ala$^5$, D-Trp$^8$, D-Cys$^{14}$-Somatostatin);

(ii)    X is H-D-Ala-Gly; Y is D-Ala, and Z is Cha (i.e. D-Ala$^1$, D-Ala$^5$, D-Trp$^8$, Cha$^{11}$, D-Cys$^{14}$-Somatostatin);

(iii)   X is H-D-Ser-Gly; Y is Ala, and Z is Phe (i.e. D-Ser$^1$, Ala$^5$, D-Trp$^8$, D-Cys$^{14}$-Somatostatin);

(iv)    X is H-D-Ser-Gly; Y is Ala, and Z is D-Phe (i.e. D-Ser$^1$, Ala$^5$, D-Trp$^8$, D-Phe$^{11}$, D-Cys$^{14}$-Somatostatin)

(v)     X is H-D-Ser-Gly; Y is Ala; and Z is Cha (i.e. D-Ser$^1$, Ala$^5$, D-Trp$^8$, Cha$^{11}$, D-Cys$^{14}$-Somatostatin)

Compounds (i), (ii), and (iv), when tested in a dog stimulated with L-alanine, did not inhibit secretion of insulin or glucagon.

The invention also contemplates the linear form (IV) of the tetradecapeptides of Formula III:

X-Cys-Lys-Y-Phe-Phe-D-Trp-Lys-Thr-Z-Thr-Ser-D-Cys-OH

IV

or a non-toxic acid addition salt thereof; wherein X, Y and Z have the meanings hereinbefore defined with respect to Formula III. The linear peptides defined by Formula IV are precursors in the preparation of the peptides of Formula III. In the cyclic form (III), the two cysteine residues ($Cys^3$ and $D-Cys^{14}$) are linked by means of a disulfide bond formed between the side chain sulfhydyl functions to form cystine. In the linear form, the two cysteine residues are not linked and the side-chain sulfhydyl functions are free.

The invention also contemplates the protected peptides of Formula V:

$$X^1-Cys(R^1)-Lys(R^2)-Y^1-Phe-Phe-D-Trp(R^3)-Lys(R^2)$$

$$W-D-Cys(R^1)-Ser(R^4)-Thr(R^4)-Z^1-Thr(R^4)$$

V

wherein:

$X^1$ is R-Ala-Gly, R-Ala-D-Ala, R-D-Ala-Gly, R-D-Val-Gly, R-Ala-D-Val, R-Ala-D-Ser($R^4$), or R-D-Ser($R^4$)-Gly, wherein R is H or an α-amino protecting group;

$Y^1$ is Ala or D-Ala Z is Phe, D-Phe, or Cha;

$R^1$ is a sulfhydryl protecting group;

$R^2$ is an ε-amino protecting group;

$R^3$ is hydrogen or formyl;

$R^4$ is a hydroxyl protecting group; and

W is -OH, -OCH$_3$, or -O-CH$_2$-[polystyrene resin]; and, when R is H, the non-toxic pharmaceutically acceptable acid addition salts thereof.

The peptides of Formula V are intermediates in the synthesis or the peptides of Formula III and IV.

In the synthesis of the peptides of Formula III, the peptide chain is built stepwise by the sequential coupling of individual amino acids commencing from the C-terminal end of the chain. During each coupling, the amino acids must be protected at the α-amino group and, if necessary, at reactive side-chain functional groups to prevent the formation of undesirable side products. In the peptides of Formula V, the protecting groups represented by R, R$^1$, R$^2$, and R$^4$ were employed to block reactive α-amino or side-chain groups in the individual amino acids during their incorporation into the peptide chain. The protecting groups represented by R, R$^1$, R$^2$, and R$^4$ can, therefore, be any group known in the art to be useful for the stepwise synthesis of polypeptides. Such groups are well-known, and the selection of a particular protecting group and its method of use will be readily apparent to a peptide chemist of ordinary skill. Illustrative examples of protecting groups for R, R$^1$, R$^2$ and R$^4$ are set forth below:

A. The α-amino protecting groups contemplated for R are well recognized by those of ordinary skill in the peptide art. Many of these are detailed in the treatise _Protective Groups in Organic Chemistry_,

J.F.W. McOmie, Ed., Plenum Press, N.Y. 1973, in Chapter 2, authored by J.W. Barton. For an α-amino group present in the N-terminal amino acid residue, R may be: (a) acyl-type groups, such as formyl, trifluoracetyl, phthalyl, p-toluenesulfonyl (tosyl), benzenesulfonyl, and nitrophenylsulfenyl; (b) aromatic urethane-type groups, such as benzyloxycarbonyl and substituted benzyloxycarbonyl, for example: p-chlorobenzyloxycarbonyl, p-bromobenzyloxycarbonyl, p-nitrobenzyloxycarbonyl, p-methoxybenzyloxycarbonyl, o-chloro-benzyloxycarbonyl, 2,4-dichlorobenzyl-oxycarbonyl, and 2,6-dichlorobenzyloxy-carbonyl; (c) aliphatic urethane type groups such as t-butyloxycarbonyl, t-amyloxycarbonyl, isopropyloxycarbonyl, 2-(p-biphenylyl)isopropyloxycarbonyl, and allyloxycarbonyl; (d) cycloalkyl urethane-type groups such as cyclopentyloxycar-bonyl, cyclohexyloxycarbonyl, cycloheptyl-oxycarbonyl, and adamantyloxycarbonyl; (e) thio urethane-type groups such as phenylthiocarbonyl; (f) alkyl-type groups such as triphenylmethyl; or (g) trialkylsilane groups, such as trimethylsilane. The preferred α-amino protecting group defined by R is t-butyloxycarbonyl (BOC).

B. For the sulfhydryl group present in cysteine, $R^1$ may be benzyl and substituted benzyl (e.g. 3,4-dimethylbenzyl, p-methoxybenzyl, p-methylbenzyl, p-chlorobenzyl, p-nitrobenzyl), trityl, benzyloxycarbonyl, benzhydryl, p-methoxybenzyloxycarbonyl, benzylthiomethyl, ethylcarbamoyl, thioethyl, tetrahydropyranyl, acetamidomethyl, and benzoyl. For additional groups, see, for example, Houben-Weyl, *Methodes der Organischen Chemie*, "Synthese von Peptiden", Vols. 15/1 and 15/2, (1974), Stuttgart, Germany. The preferred sulfhydryl protecting group defined by $R^1$ is p-methoxybenzyl (MBzl).

C. For the ε-amino protecting group present in lysine, $R^2$ may be one of the groups mentioned hereinabove for the protection of an α-amino group. Typical groups include for example, benzyloxycarbonyl, p-chlorobenzyloxycarbonyl, p-bromobenzyloxycarbonyl, o-chlorobenzyloxycarbonyl, 2,6-dichlorobenzyloxycarbonyl, 2,4-dichlorobenzyloxycarbonyl, o-bromobenzyloxycarbonyl, p-nitrobenzyloxycarbonyl t-butyloxycarbonyl, isopropyloxycarbonyl, t-amyloxycarbonyl, cyclopentyloxycarbonyl, cyclohexyloxycarbonyl, cycloheptyloxycarbonyl, adamantyloxycarbonyl, and p-toluenesulfonyl. The

preferred ε-amino protecting group defined by $R^2$ is o-chlorobenzyloxy-carbonyl (ClBzl).

D.  For the hydroxyl group of serine or threonine, $R^4$ may be $C_1$-$C_4$ alkyl (e.g. methyl, ethyl, t-butyl), benzyl, substituted benzyl (e.g. p-methoxybenzyl, p-nitrobenzyl, p-chlorobenzyl, o-chlorobenzyl), $C_1$-$C_3$ alkanoyl (e.g. formyl, acetyl, propionyl), triphenylmethyl, or benzoyl.  The preferred hydroxyl protecting group defined by $R^4$ is benzyl (Bzl).

The group $R^3$ represents either hydrogen or formyl substituted on the nitrogen of the indole ring of tryptophan.  The use of formyl as a protecting group is optional.  $R^3$ is preferably hydrogen.

In Formula V, when W represents "-O-$CH_2$-[polystyrene resin]" the peptide chain is attached to the polystyrene resin by means of an ester linkage, (-D-Cys-O-$CH_2$-) formed between the carboxyl group of the C-terminal D-cysteine moiety and one of the methylene groups present on the resin matrix as sites for such attachment.  The polystyrene resin is a styrene polymer which is cross linked by the addition of about 0.5 to about 3% divinylbenzene and which is chloromethylated or hydroxymethylated to provide sites for ester formation.  An example of a hydroxymethylated resin is described by Bodanszky et al. Chem. Ind. (London) 38, 1597-98 (1966).  A chloromethylated polystyrene resin is commercially available from Lab System, Inc., San Mateo, California.  The resin is also described by Stewart et al. Solid Phase Peptide Synthesis, Freeman and Co., San Francisco, California, pp. 1-6.

The tetradecapeptides of Formula III can be made either by classical (solution) methods or by the solid phase method using techniques generally known in the art for forming peptide bonds.  The peptide can be assembled either by coupling each amino acid separately or by coupling appropriate pre-formed peptide segments in the desired order.

The preferred method of preparation of the peptides of Formula III, and of the intermediates of Formula IV and V is by the solid phase technique in which the amino acid sequence is built sequentially from an initial, insoluble resin-supported C-terminal amino acid.  Techniques for the solid phase method are described by J. Stewart et al., Solid Phase Peptide Synthesis, Freeman and Co., San Francisco, 1969.

In general, in the solid phase method, the amino acid corresponding to the C-terminal amino acid residue of the desired peptide is anchored to an insoluble resin support, and the peptide chain is then formed beginning at the resin-supported C-terminal amino acid by introducing the individual amino acids one at a time until the desired amino acid sequence is achieved.  Alternatively, small peptide fragments can be prepared and introduced into the peptide chain in the desired order.  The peptide chain remains attached to the resin throughout the synthesis, and, upon completion of the chain, the peptide is cleaved from the resin.

The amino acids are coupled using techniques well-known in the art for the formation of a peptide bond. One method is to convert the amino acid to a derivative that will render the carboxyl group more reactive to reaction with the free N-terminal amino group of the peptide fragment. For example, the amino acid can be converted to a mixed anhydride by reaction of a protected amino acid with ethyl chloroformate, phenyl chloroformate, sec-butyl chloroformate, isobutylchloroformate, pivaloyl chloride, or like acid chlorides. Alternatively, the amino acid can be converted to an active ester such as a 2,4,5-trichlorophenyl ester, a pentachlorophenyl ester, a p-nitrophenyl ester, an ester formed from N-hydroxysuccinimide, or an ester formed from 1-hydroxybenzotriazole.

Another method is to perform the coupling reaction with a suitable coupling agent, such as N,N'-dicyclohexylcarbodimide (DCC) or N,N'-diisopropylcarbodiimide (DIC). Other appropriate coupling agents will be apparent to those skilled in the art. [See Schroder and Lubke, The Peptides, Academic Press, 1965, Chapter III.]

It should be recognized that the α-amino group of each amino acid employed in the peptide synthesis must be protected during the coupling reaction to prevent side reactions involving the reactive α-amino function. It should also be recognized that certain amino acids contain reactive side-chain functional groups (e.g. sulfhydryl, ε-amino, and hydroxyl), and such functional groups must also be protected both during the initial coupling of the amino acid containing the side-chain group and during the coupling of subsequent amino acids.

In selecting a particular protecting group the following conditions must be observed: An α-amino protecting group must: (1) be stable and render the α-amino function inert under the conditions employed in the coupling reaction, and (2) must be readily removable after the coupling reaction under conditions that will not remove the side chain protecting groups or alter the structure of the peptide fragment. A side chain protecting group must: (1) be stable and render the side chain functional group inert under the conditions employed in the coupling reaction, (2) be stable under the conditions employed in removing the α-amino protecting group, and (3) be readily removable upon completion of the desired amino acid sequence under reaction conditions that will not alter the structure of the peptide chain.

It will be apparent to those skilled in the art that the protecting groups known in the art to be useful for peptide synthesis will vary in their reactivity towards the acidic agents employed for their removal. For example, certain protecting groups, such as triphenylmethyl and 2-(p-biphenylyl)-isopropyloxycarbonyl are very labile and can be cleaved under mild acid conditions. Other protecting groups, such as t-butyloxycarbonyl, t-amyloxycarbonyl, adamantyloxycarbonyl, and p-methoxybenzyloxycarbonyl are less labile and require moderately stong acids (such as trifluoroacetic, hydrochloric, or borontrifluoride in acetic acid) for their removal. Still other protecting groups, such as benzyloxycarbonyl, halobenzyloxycarbonyl, p-nitrobenzyloxycarbonyl, cycloalkoxycarbonyl, and isopropyloxycarbonyl, are even less labile and require strong acids, such as hydrogen fluoride, hydrogen bromide, boron trifluoroacetate in trifluoroacetic acid for their removal.

Upon completion of the desired peptide sequence, the protected peptide must be cleaved from the resin support, and all protecting groups must be removed. The cleavage reaction and removal of the protecting groups may be accomplished simultaneously or stepwise. When the resin support is a chloromethylated polystyrene resin, the bond anchoring the peptide to the resin is an ester linkage formed between the free carboxyl group of the C-terminal cysteine moiety and one of the many chloromethyl groups present on the resin matrix. It will be recognized that the anchoring bond can be cleaved by reagents which are known to be capable of breaking an ester linkage and of penetrating the resin matrix. One especially convenient method is by treatment with liquid hydrogen fluoride. This reagent will not only cleave the peptide from the resin but will also remove all protecting groups. Hence, use of this reagent will directly afford the fully deprotected linear form of the peptide. When it is desired to cleave the peptide without removing protecting groups, the protected peptide-resin can undergo methanolysis to give the protected linear peptide in which the C-terminal carboxyl group is methylated. The methyl ester can then be hydrolyzed under mild, alkaline conditions to give the free C-terminal carboxyl. The protecting groups on the peptide chain can then be removed by treatment with a strong acid, such as liquid hydrogen fluoride. A particularly useful technique for methanolysis is that of G. Moore et al., Peptides, Proc. 5th Amer. Pept. Symp., M. Goodman and J. Meinhofer, Eds., John Wiley, N.Y., 1977, pp. 518-521 in which the protected peptide-resin is treated with methanol and potassium cyanide in the presence of crown ether.

Another method for cleaving the protected peptide from the resin is by ammonolysis or by treatment with hydrazine. The resulting C-terminal amide or hydrazide can be hydrolyzed to the free C-terminal carboxyl, and the protecting groups can be removed conventionally.

It will also be recognized that the protecting group present on the N-terminal $\alpha$-amino group may be removed preferentially either before or after the protected peptide is cleaved from the resin support.

Upon cleavage from the resin and the removal of all protecting groups, the product obtained is in the form of the linear tetradecapeptide, Formula IV. The linear tetradecapeptide can be cyclized to the final cyclic tetradecapeptide (III) by means of an oxidizing agent capable of converting the two sulfhydyl groups of $Cys^3$ and $D-Cys^{14}$ to the disulfide bond. Both exposure to air or treatment with potassium ferricyanide may be used to effect such oxidation. When air is employed, the pH of the medium should be about 2.5 to about 9.0 and preferably about 7.0 to 7.6 and the concentration of the peptide should not be above 0.4 mg/ml. A concentration of about 50 µg/ml is preferred.

For pharmacological purposes, the peptides of Formula III can be administered in the form of an acid addition salt prepared by reaction with an appropriate organic or inorganic acid which is non-toxic and acceptable for pharmaceutical purposes. Suitable acids are well known in the art. Illustrative of such acids are hydrochloric, hydrobromic, sulfuric, sulfonic, tartaric, fumaric, glycolic, succinic, malonic, citric, maleic, acetic, phosphoric, benzoic, ascorbic, nitric, p-toluenesulfonic, benzenesulfonic, naphthalenesulfonic, propionic, and the like. Acetic acid is preferred.

The preferred method for the solid phase synthesis of the peptides of Formula III and the intermediates thereto, is illustrated by the Examples. In this method, α-amino and sulfhydyl protected D-cysteine (BOC-D-Cys(MBzl)-OH) is first attached to chloromethylated polystyrene resin according to the method of B. Gisin Helv. Chim. Acta., 56, 1476 (1173) wherein the cesium salt of the protected D-cysteine is reacted with the chloromethylated polystyrene resin in dimethylformamide. The t-butyloxycarbonyl protecting group is then removed by treatment with trifluoroacetic acid in chloroform-methylene chloride. Individual protected amino acids are then coupled sequentially beginning at the resin-supported C-terminal D-cysteine until the desired tetradecapeptide is achieved. Throughout the synthesis, N,N'-dicyclohexylcarbodiimide is used as the coupling agent, and t-butyloxycarbonyl (BOC) is used as the α-amino protecting group. The side chain protecting groups are: p-methoxybenzyl (MBzl) for the sulfhydyl group of cysteine; o-chloro-benzyloxycarbonyl (Clz) for the ε-amino of lysine; and benzyl (Bzl) for the hydroxyl of serine and threonine. Trifluoroacetic acid in methylenechloride is employed to remove preferentially the t-butyloxycarbonyl protecting group. After each deprotection, the side-chain protected peptide is neutralized with triethylamine.

Upon completion of the desired amino acid sequence, the resulting tetradecapeptide is deprotected and removed from the polystyrene resin by treatment with liquid hydrogen fluoride in the presence of anisole and ethylmercaptan. The resulting linear tetradecapeptide (IV) is readily converted to the cyclic tetradecapeptide (III) by exposure of a solution of the linear tetradecapeptide (IV) to atmospheric oxygen. The cyclic tetradecapeptide is purified by chomotography.

For pharmacological use, the tetradecapeptides of Formula III may be administered alone on in combination with pharmaceutically acceptable carriers or excipients. Suitable pharmaceutical carriers will be apparent to those skilled in the art. Administration may be orally or parenterally by methods conventional in the art of medicine.

The method of making and using the peptides of the invention are illustrated in the following Examples. In the following examples the following symbols are used:

conc. = concentrated

t-BuOH = t-butyl alcohol

t-AmOH = t-amyl alcohol

DMF = dimethylformamide

STARTING MATERIALS

### Example A

N-t-Butyloxycarbonyl-D-(S-p-methoxybenzyl)cysteine hydroxymethyl-polystyrene resin ester

Chloromethylated polystryene resin is esterified by the method of F. Gisin <u>Helv. Chim. Acta.</u> <u>56</u>, 1976 (1973).

A solution of the cesium salt of t-butyloxycarbonyl-D-(S-p-methoxybenzyl)cysteine (24.95 mmoles) in DMF (500 ml.) is stirred with chloromethylated polystyrene resin (Lab Systems, Inc.) (50 g.; 0.75 mmole Cl/g.) at room temperature for four days. The resin is separated by filtration and washed with 85% DMF - 15% water and then with DMF alone. This wash sequence is repeated two additional times. After two more washings with DMF, the resin is suspended in DMF (500 ml.), and the suspension is stirred with cesium acetate (8 g.; 41.7 mmole) at room temperature for three days. The resin is separated by filtration and washed with 85% DMF - 15% water and with DMF alone. This sequence is repeated two additional times. The resin is finally washed with chloroform and is suspended in chloroform contained in a separatory funnel. Fines are removed by drawing off liquid. This separation is repeated three additional times. The resin is collected by filtration and washed successively with 95% ethanol, benzene, and 95% ethanol. The latter two washes are repeated two additional times. The resin is dried overnight in vacuo at 40°C. to give 58.1 g. of the title product. A portion of the resin is assayed for cysteine after hydrolysis using a 1:1 mixture of conc. hydrochloric acid-dioxane in the presence of a small amount of dimethylsulfoxide.
Found:   0.237 mmole cysteine per g. of resin.

## Example B

N-t-Butyloxycarbonyl-D-alanyl-glycyl-L-(S-p-methoxy-benzyl)cysteinyl-L-(N$^\epsilon$-o-chlorobenzyloxycarbonyl)lysyl-D-alanyl-L-phenylalanyl-L-phenylalanyl-D-tryptophyl-L-(N$^\epsilon$-o-chlorobenzyloxycarbonyl)lysyl-L-(O-benzyl)threonyl-L-phenylalanyl-L-(O-benzyl)threonyl-L-(O-benzyl)seryl-D-(S-p-methoxybenzyl)cysteine hydroxymethyl-polystyrene resin ester

N-t-Butyloxycarbonyl-D-(S-p-methoxybenzyl)-cysteine hydroxymethyl-polystyrene resin ester (7.0 g.), as prepared in Example A, is placed in the reaction chamber of a Beckman 990 peptide synthesizer and is treated according to Schedule A (set forth below), N-t-butyloxycarbonyl-L-(O-benzyl)serine being employed as the amino acid added in Step 11 thereof. After the final methylene chloride wash (Step 18), the product is washed three times with dimethylformamide (DMF) and is re-coupled by following Step 11 of Schedule A. The product is then washed three times with DMF and is retreated by following Steps 12-18 of Schedule A.

In a similar manner, the following protected amino acids are incorporated sequentially into the peptide resin:

N-t-butyloxycarbonyl-L-(O-benzyl)threonine
N-t-butyloxycarbonyl-L-phenylalanine
N-t-butyloxycarbonyl-L-(O-benzyl)threonine
N-t-butyloxycarbonyl-L-(N$^\varepsilon$-o-chlorobenzyloxy-carbonyl)lysine
N-t-butyloxycarbonyl-D-tryptophane
N-t-butyloxycarbonyl-L-phenylalanine
N-t-butyloxycarbonyl-L-phenylalanine
N-t-butyloxycarbonyl-D-alanine*
N-t-butyloxycarbonyl-L-(N$^\varepsilon$-o-chlorobenzyloxy-carbonyl)lysine
N-t-butyloxycarbonyl-L-(S-p-methoxybenzyl)-cysteine
N-t-butyloxycarbonyl-glycine
N-t-butyloxycarbonyl-D-alanine*

* Steps 10-18 of Schedule A are not repeated during the incorporation of BOC-Ala.

After incorporation of the N-terminal amino acid residue (D-alanine), the peptide resin is dried in vacuo. An amino acid analysis (obtained by refluxing a portion of the peptide for 72 hours in conc. hydrochloric acid-dioxane, 1:1) gives the following results, lysine being employed as the standard: 2 Thr, 2.24; Ser, 1.01; Gly, 1.01; 2 Ala, 2.10; 3 Phe, 2.97; 2 Lys, 2.00.

SCHEDULE A   [Protocol for the removal of the t-butyloxycarbonyl α-amino protecting group and the coupling of the amino acid to the peptide-resin]

1. Wash with $CHCl_3$, three times.

2.  To remove the t-butyloxycarbonyl α-amino protecting group, treat with a mixture of trifluoroacetic acid (28.8%), $CH_2Cl_2$ (17.5%), 47.9% $CHCl_3$, and triethylsilane (5.8%) for twenty minutes.  Repeat one time.

3.  Wash with $CHCl_3$ two times.

4.  Wash with $CH_2Cl_2$, one time.

5.  Wash with 90% t-BuOH-10% t-AmOH, three times.

6.  Wash with $CH_2Cl_2$, three times.

7.  For neutralization, treat with 3% triethylamine in $CH_2Cl_2$, three times.

8.  Wash with $CH_2Cl_2$, three times.

9.  Wash with 90% t-BuOH -10% t-AmOH, three times.

10.  Wash with $CH_2Cl_2$, three times.

11.  To couple the amino acid, treat with the protected amino acid (1.0 mmole/g. resin) and N,N'-dicyclohexylcarbodiimide (1.0 mmole/g. resin) in $CH_2Cl_2$.  Allow two-hour reaction time.

12.  Wash with $CH_2Cl_2$, three times.

13.  Wash with 90% t-BuOH-10% t-AmOH.

14.  Wash with $CH_2Cl_2$, three times.

15.  For neutralization, treat with 3% triethylamine in $CH_2Cl_2$, three times.

16. Wash with $CH_2Cl_2$, three times.

17. Wash with 90% t-BuOH-10% t-AmOH, three times.

18. Wash with $CH_2Cl_2$, three times.

The volume of solvent employed for each step is eight ml./g. of resin. Unless otherwise noted, the contact time for each step is three minutes.

### Example C

N-t-Butyloxycarbonyl-D-alanyl-glycyl-L-(S-p-methoxybenzyl)cysteinyl-L-(N$^\varepsilon$-o-chlorobenzyloxycarbonyl)lysyl-D-alanyl-L-phenylalanyl-L-phenylalanyl-D-tryptophyl-L-(N$^\varepsilon$-o-chlorobenzyloxycarbonyl)-lysyl-L-(O-benzyl)threonyl-L-cyclohexylalanyl-L-(O-benzyl)threonyl-L-(O-benzyl)seryl-D-(S-p-methoxybenzyl)cysteine.hydroxymethylpolystyrene resin ester

N-t-Butyloxycarbonyl-D-(S-p-methoxybenzyl) cysteine hydroxymethyl-polystyrene resin ester (7.0 g.), as prepared in Example A, is placed in the reaction chamber of a Beckman 990 peptide synthesizer and is treated according to Schedule A (set forth in Example B), N-t-butyloxycarbonyl-L-(O-benzyl)serine being employed as the amino acid added in Step 11 thereof. After the final methylene chloride wash (Step 18), the product is washed three times with DMF and is re-coupled by following Step 11 of Schedule A. The product is then washed three times with DMF and is retreated by following Steps 12-18 of Schedule A.

In a similar manner, the following protected amino acids are incorporated sequentially into the peptide resin:

N-t-butyloxycarbonyl-L-(O-benzyl)threonine N-t-butyloxycarbonyl-L-cyclohexylalanine N-t-butyloxycarbonyl-L-(O-benzyl)threonine N-t-butyloxycarbonyl-L-(N$^\epsilon$-o-chlorobenzyloxy-carbonyl)lysine

N-t-butyloxycarbonyl-D-tryptophane N-t-butyloxycarbonyl-L-phenylalanine

N-t-butyloxycarbonyl-L-phenylalanine*

N-t-butyloxycarbonyl-D-alanine**

N-t-butyloxycarbonyl-L-(N$^\epsilon$-o-chlorobenzyloxy-carbonyl)lysine

N-t-butyloxycarbonyl-L-(S-p-methoxybenzyl)-cysteine

N-t-butyloxycarbonyl-glycine

N-t-butyloxycarbonyl-D-alanine

* After introduction of BOC-Phe at position 6, the peptide-resin is divided into two equal portions and BOC-D-Ala and the remaining amino acid residues are introduced using only one portion.

** Steps 10-18 of Schedule A are not repeated during the incorporation of BOC-Ala.

After incorporation of the N-terminal amino acid residue (D-alanine), the peptide resin is dried in vacuo. An amino acid analysis (obtained by refluxing a portion of the peptide for 72 hours in conc. hydrochloric acid-dioxane, 1:1) gives the following results, lysine being employed as the standard:

2 Thr, 2.26; Ser, 1.03; Gly, 1.04; 2 Ala, 2.18;
2 Phe, 2.14; 2 Lys, 2.00; Cha, 1.14.

## Example D

N-t-Butyloxycarbonyl-D-seryl-glycyl-L-(S-p-methoxy-benzyl)cysteinyl-L-(Nᵉ-o-chlorobenzyloxycarbonyl)-lysyl-L-alanyl-L-phenylalanyl-L-phenylalanyl-D-tryptophyl-L-(Nᵉ-o-chlorobenzyloxycarbonyl)lysyl-L-(O-benzyl)threonyl-L-phenylalanyl-L-(O-benzyl)-threonyl-L-(O-benzyl)seryl-D-(S-p-methoxybenzyl)-cysteine hydroxymethylpolystyrene resin ester

N-t-Butyloxycarbonyl-D-(S-p-methoxybenzyl)cysteine hydroxymethyl-polystyrene resin ester (5.5 g.), as prepared in Example A, is placed in the reaction chamber of a Beckman 990 peptide synthesizer and is treated according to Schedule A (set forth in Example B), N-t-butyloxycarbonyl-L-(O-benzyl)serine being employed as the amino acid added in Step 11 thereof. After the final methylene chloride wash (Step 18), the product is washed three times with DMF and is re-coupled by following Step 11 of Schedule A. The product is then washed three times with DMF and is retreated by following Steps 12-18 of Schedule A.

In a similar manner, the following protected amino acids are incorporated sequentially into the peptide resin:

N-t-butyloxycarbonyl-L-(O-benzyl)threonine N-t-butyloxycarbonyl-L-phenylalanine

N-t-butyloxycarbonyl-L-(O-benzyl)threonine N-t-butyloxycarbonyl-L-(Nᵉ-o-chlorobenzyloxy-carbonyl)lysine

N-$\underline{t}$-butyloxycarbonyl-D-tryptophane

N-$\underline{t}$-butyloxycarbonyl-L-phenylalanine

N-$\underline{t}$-butyloxycarbonyl-L-phenylalanine

N-$\underline{t}$-butyloxycarbonyl-L-alanine*

N-$\underline{t}$-butyloxycarbonyl-L-(N$^{\varepsilon}$-$\underline{o}$-chlorobenzyloxy-carbonyl)lysine

N-$\underline{t}$-butyloxycarbonyl-L-(S-$\underline{p}$-methoxybenzyl)-cysteine

N-$\underline{t}$-butyloxycarbonyl-glycine

N-$\underline{t}$-butyloxycarbonyl-D-serine

* Steps 10-18 of Schedule A are not repeated during the incorporation of BOC-Ala.

After incorporation of the N-terminal amino acid residue (D-valine), the peptide resin is dried $\underline{in}$ $\underline{vacuo}$. An amino acid analysis (obtained by refluxing a portion of the peptide for 72 hours in conc. hydrochloric acid-dioxane, 1:1) gives the following results, lysine being employed as the standard:

2 Thr, 1.80; 2 Ser, 1.52; Gly, 1.02; Ala, 1.07; 3 Phe, 2.88; 2 Lys, 2.00.

## Example E

N-$\underline{t}$-Butyloxycarbonyl-D-seryl-glycyl-L-(S-$\underline{p}$-methoxy-benzyl)cysteinyl-L-(N$^{\varepsilon}$-$\underline{o}$-chlorobenzyloxycarbonyl)lysyl-L-alanyl-L-phenylalanyl-L-phenylalanyl-D-tryptophyl-L-(N$^{\varepsilon}$-$\underline{o}$-chlorobenzyloxycarbonyl)lysyl-L-(O-benzyl)threonyl-D-phenylalanyl-L-(O-benzyl)threonyl-L-(O-benzyl)seryl-D-(S-$\underline{p}$-methoxybenzyl)cysteine hydroxymethylpolystyrene resin ester

N-$\underline{t}$-Butyloxycarbonyl-D-(S-$\underline{p}$-methoxybenzyl)cysteine hydroxymethyl-polystyrene resin ester (3.5 g.), as prepared in Example A, is placed in the reaction chamber of a Beckman 990 peptide synthesizer and is treated according to Schedule A (set forth in Example B), N-t-butyloxycarbonyl-L-(O-benzyl)serine being employed as the amino acid added in Step 11 thereof. After the final methylene chloride wash (Step 18), the product is washed three times with DMF and is re-coupled by following Step 11 of Schedule A. The product is then washed three times with DMF and is retreated by following Steps 12-18 of Schedule A.

In a similar manner, the following protected amino acids are incorporated sequentially into the peptide resin:

N-t-butyloxycarbonyl-L-(O-benzyl)threonine
N-t-butyloxycarbonyl-D-phenylalanine
N-t-butyloxycarbonyl-L-(O-benzyl)threonine
N-t-butyloxycarbonyl-L-(N$^{\varepsilon}$-o-chlorobenzyloxy-carbonyl)lysine
N-t-butyloxycarbonyl-D-tryptophane
N-t-butyloxycarbonyl-L-phenylalanine
N-t-butyloxycarbonyl-L-phenylalanine
N-t-butyloxycarbonyl-L-alanine*
N-t-butyloxycarbonyl-L-(N$^{\varepsilon}$-o-chlorobenzyloxy-carbonyl)lysine
N-t-butyloxycarbonyl-L-(S-p-methoxybenzyl)-cysteine
N-t-butyloxycarbonyl-glycine
N-t-butyloxycarbonyl-D-serine

* Steps 10-18 of Schedule A are not repeated during the incorporation of BOC-Ala.

After incorporation of the N-terminal amino acid residue (D-valine), the peptide resin is dried _in vacuo_. An amino acid analysis (obtained by refluxing a portion of the peptide for 72 hours in conc. hydrochloric acid-dioxane, 1:1) gives the following results, lysine being employed as the standard: 2 Thr, 2.00; 2 Ser, 1.72; Gly, 1.25; Ala, 1.09; 3 Phe, 2.97; 2 Lys, 2.00.

### Example F

N-$\underline{t}$-Butyloxycarbonyl-D-seryl-glycyl-L-(S-$\underline{p}$-methoxybenzyl)cysteinyl-L-(N$^{\varepsilon}$-$\underline{o}$-chlorobenzyloxycarbonyl)-lysyl-L-alanyl-L-phenylalanyl-L-phenylalanyl-D-tryptophyl-L-(N$^{\varepsilon}$-$\underline{o}$-chlorobenzyloxycarbonyl)lysyl-L-(O-benzyl)threonyl-L-cyclohexylalanyl-L-(O-benzyl)-threonyl-L-(O-benzyl)seryl-D-(S-$\underline{p}$-methoxybenzyl)cysteine hydroxymethylpolystyrene resin ester

N-$\underline{t}$-Butyloxycarbonyl-D-(S-$\underline{p}$-methoxybenzyl)cysteine hydroxymethyl-polystyrene resin ester (7.0 g.), as prepared in Example A, is placed in the reaction chamber of a Beckman 990 peptide synthesizer and is treated according to Schedule A (set forth in Example B), N-$\underline{t}$-butyloxycarbonyl-L-(O-benzyl)serine being employed as the amino acid added in Step 11 thereof. After the final methylene chloride wash (Step 18), the product is washed three times with dimethylformamide (DMF) and is re-coupled by following Step 11 of Schedule A. The product is then washed three times with DMF and is retreated by following Steps 12-18 of Schedule A.

0022621

In a similar manner, the following protected amino acids are incorporated sequentially into the peptide resin:

N-$\underline{t}$-butyloxycarbonyl-L-(O-benzyl)threonine

N-$\underline{t}$-butyloxycarbonyl-L-cyclohexylalanine

N-$\underline{t}$-butyloxycarbonyl-L-(O-benzyl)threonine

N-$\underline{t}$-butyloxycarbonyl-L-(N$^{\varepsilon}$-$\underline{o}$-chlorobenzyloxy-carbonyl)lysine

N-$\underline{t}$-butyloxycarbonyl-D-tryptophane

N-$\underline{t}$-butyloxycarbonyl-L-phenylalanine

N-$\underline{t}$-butyloxycarbonyl-L-phenylalanine*

N-$\underline{t}$-butyloxycarbonyl-L-alanine**

N-$\underline{t}$-butyloxycarbonyl-L-(N$^{\varepsilon}$-$\underline{o}$-chlorobenzyloxy-carbonyl)lysine

N-$\underline{t}$-butyloxycarbonyl-L-(S-$\underline{p}$-methoxybenzyl)-cysteine

N-$\underline{t}$-butyloxycarbonyl-glycine

N-$\underline{t}$-butyloxycarbonyl-D-serine

\* After introduction of BOC-Phe at position 6, the peptide-resin is divided into two equal portions, and BOC-D-Ala and the remaining amino acid residues are introduced using only one portion.

\*\* Steps 10-18 of Schedule A are not repeated during the incorporation of BOC-Ala.

After incorporation of the N-terminal amino acid residue (D-serine), the peptide resin is dried _in vacuo_. An amino acid analysis (obtained by refluxing a portion of the peptide for 72 hours in conc. hydrochloric acid-dioxane, 1:1) gives the following results, lysine being employed as the standard:
2 Thr, 2.32; 2 Ser, 2.48; Gly, 1.45; Ala, 1.26; 2 Phe, 2.06; 2 Lys, 2.00; Cha, 1.23.

## FINAL PRODUCTS

### Example 1

D-Alanyl-glycyl-L-cysteinyl-L-lysyl-D-alanyl-L-phenylalanyl-L-phenylalanyl-D-tryptophyl-L-lysyl-L-threonyl-L-phenylalanyl-L-threonyl-L-seryl-D-cysteine.

The protected peptido-resin prepared in Example B (5.58 g., at a substitution level of 0.178 mmole/g. resin) is mixed with anisole (10.2 ml.) and ethyl mercaptan (10.2 ml). The mixture is cooled with liquid nitrogen and liquid hydrogen fluoride (145 ml.) is added by distillation. The mixture is then brought to 0°C. and is stirred for two hours. Removal of hydrogen fluoride by distillation gives a residue to which is added ether at 0°C. The solid is collected, washed with ether, and dried. The peptide is separated from the resin by extracting the solid with 0.2M acetic acid and 50% acetic acid. The extract is filtered through glass fiber filter paper to remove suspended insoluble material. The filtrate is chromatographed through a Sephadex G-25 Fine column under the following conditions--solvent: degassed 0.2M acetic acid; column size: 7.5 X 225 cm; temperature: 26°C; flow rate: 1317 ml/hour; fraction volume: 22.0 ml. A 6637 ml. sample is collected in large flasks and discarded before individual fractions are collected.

A plot of absorbance at 280 mμ of each fraction versus fraction number shows a large peak with a front-side shoulder. UV spectrographic analysis indicates that the fractions represented by the broad peak contain the desired product. Hence, fractions 52-85 (7754-8502 ml, peak 8229 ml) are combined. UV spectrographic analysis of a sample of the combined fractions indicates that 514 mg. of the product is obtained. Free sulfhydryl content: 98.0% of theoretical (by Ellman titration)

## Example 2

### D-Alanyl-glycyl-L-cysteinyl-L-lysyl-D-alanyl-L-phenylalanyl-L-phenylalanyl-D-tryptophyl-L-lysyl-L-threonyl-L-phenylalanyl-L-threonyl-L-seryl-D-cysteine cyclic (3 -> 14) disulfide.

The linear peptide prepared in Example 1 is air oxidized to the corresponding cyclic peptide by the following procedure:

The combined fractions obtained in Example 1 (748 ml, theoretically containing 514 mg. of peptide) are diluted with 9532 ml of distilled water to achieve a final solution having a concentration of 50 µg/ml. Sufficient concentrated ammonium hydroxide is added to bring the pH to 6.7. The solution is then stirred at room temperature in the dark for 19 hours at which time Ellman titration of an aliquot indicates complete oxidation.

The solution is concentrated in vacuo to a volume of about 110 ml. Glacial acetic acid (110 ml) is added and the solution is desalted by chomatography on a Sephadex G-25 Fine Column under the following conditions -- solvent: degassed 50% acetic acid; column size: 7.5 X 210 cm; temperature: 26°C; flow rate: 1038 ml/hour; fraction volume: 22.5 ml.

A plot of absorbance at 280 mμ of each fraction versus fraction number shows two large peaks. The first represents the aggregated forms of the peptide, while the second represents monomeric material. Fractions 64-103 (4320-5220 ml.) are combined and lyophilized to dryness in the dark. The solids are dissolved in 36 ml. of water, and the solution is buffered by addition of 2 ml of 1M ammonium formate-formic acid, pH 4.25. The solution is applied to a glass column, 60 cm in length, packed with 1540 ml of reverse phase silica gel (ca 20 μm, $C_{18}$, 16.3% C), pre-equilibrated with 28% acetonitrile in 0.5M ammonium formate, formic acid, pH 4.25. The column is eluted with the same solvent, and the fractions eluting between 2740 ml. and 3230 ml are combined. The pooled sample contains 193.5 mg of peptide as determined by UV spectra. The pool showed a ratio, absorbance at 281 nm/absorbance at 249 nm, of 2.01.

The pooled sample is diluted 1:10 with distilled water, and pumped over a column (2.5 X 26 cm) packed with amberlite XAD-2 resin (Rohm and Haas Co.). The column is then washed with 250 ml of water, and finally the sample is eluted from the resin with 50% acetonitrile, 1% acetic acid in distilled water. A pool of 103 ml is collected on the basis of UV spectral monitoring of the eluant stream. It contains ca 197 mg of peptide, with a ratio (281/249 μm) of 2.02. The solution is lyophilized and the resulting solid collected. Gross weight: 204.5 mg, of which 176.5 mg is peptide on the basis of UV spectral analysis. Amino acid analysis:

Gly, 1.01; Cys, 2.35; Lys, 2.0; Ala, 2.08; Phe, 2.97; Thr, 2.00; Ser, 0.89.

The above results are expressed as ratios to Lys/2. All values are the average of 2 hydrolyses, with no added scavengers. The value for D-Trp was not determined.

## Example 3

D-Alanyl-glycyl-L-cysteinyl-L-lysyl-D-alanyl-L-phenylalanyl-L-phenylalanyl-D-tryptophyl-L-lysyl-L-threonyl-L-cyclohexylalanyl-L-threonyl-L-seryl-D-cysteine.

The protected peptido-resin prepared in Example C (5.53 g., at a substitution level of 0.176 mmole/g. resin) is mixed with anisole (10.1 ml.) and ethyl mercaptan (10.1 ml). The mixture is cooled with liquid nitrogen and liquid hydrogen fluoride (126 ml.) is added by distillation. The mixture is then brought to 0°C. and is stirred for two hours. Removal of hydrogen fluoride by distillation gives a residue to which is added ether at 0°C. The solid is collected, washed with ether, and dried. The peptide is separated from the resin by extracting the solid with 1M acetic acid and 50% acetic acid. The extract is lyophilized in the dark to dryness. Acetic acid (0.2M, 300 ml) is added to the dry material, and the suspension is warmed to effect solution. The solution is chromatographed through a Sephadex G-25 Fine column under the following conditions--solvent: degassed 0.2M acetic acid; column size: 10 X 210 cm; temperature: 26°C; flow rate: 2572 ml/hour; fraction volume: 64.3 ml. A 11,776 ml. sample is collected in large flasks and discarded before individual fractions are collected.

A plot of absorbance at 280 mμ of each fraction versus fraction number shows a broad peak with a back-side shoulder. UV spectrographic analysis indicates that the fractions represented by the broad peak contain the desired product. Hence, fractions 19-45 (12,923-14,673 ml, peak 13,798 ml) are combined. UV spectrographic analysis of a sample of the combined fractions indicates that 702 mg. of the product is obtained. Recovery: 45.0%. Free sulfhydryl content: 96.1% of theoretical (by Ellman titration)

Example 4

D-Alanyl-glycyl-L-cysteinyl-L-lysyl-D-alanyl-L-phenylalanyl-L-phenylalanyl-D-tryptophyl-L-lysyl-L-threonyl-L-cyclohexylalanyl-L-threonyl-L-seryl-D-cysteine cyclic (3 -> 14) disulfide.

The linear peptide prepared in Example 3 is air oxidized to the corresponding cyclic peptide by the following procedure:

The combined fractions obtained in Example 3 (1750 ml, theoretically containing 702 mg. of peptide) are diluted with 12,290 ml of distilled water to achieve a final solution having a concentration of 50 μg/ml. Sufficient concentrated ammonium hydroxide is added to bring the pH to 6.7. The solution is then stirred at room temperature in the dark for 41 hours at which time Ellman titration of an aliquot indicates complete oxidation.

The solution is concentrated in vacuo to a volume of about 80 ml. Glacial acetic acid (100 ml) is added and the solution is desalted by chomatography on a Sephadex G-25 Fine Column under the following conditions -- solvent:  degassed 50% acetic acid; column size: 7.5 X 211 cm; temperature: 26°C; flow rate: 113 ml/hour; fraction volume: 19.8 ml.  A 2975 ml sample is collected in large flasks and discarded before individual fractions are collected.

A plot of absorbance at 280 mµ of each fraction versus fraction number shows two large peaks. The first represents the aggregated forms of the peptide, while the second represents monomeric material. Fractions 109-128 (4988-5362 ml.) are combined and lyophilized to dryness in the dark.  The resulting solid is dissolved in degassed 0.2M acetic acid (25 ml.) and the solution is chromatographed on a Sephadex G-25 Fine column under the following conditions -- solvent: degassed 0.2M acetic acid; column size: 5 X 215 cm; temperature: 26°C; flow rate: 800 ml/hour; fraction volume: 14.65 ml.  A 2303-ml sample is collected in a large flask and discarded before individual fractions are collected.

A plot of absorbance at 280 mµ of each fraction versus fraction number shows a single peak. UV spectrographic analysis indicates that the fractions represented by the main part of this peak comprise the desired product.  Fractions 85-106 (3530-3854 ml; peak, 3667 ml) are combined and lyophilized in the dark to give the title peptide.  UV spectrographic analysis of the combined fractions before lyophilization indicated that 236.5 mg. of the product is obtained. Recovery, 33.7% (from the linear form).

Amino acid analysis:

Gly, 1.01; Cys, 2.05; Lys, 2.0; Ala, 2.15; Phe, 2.19;
Thr, 2.14; Ser, 0.85.

The above results are expressed as ratios to Lys/2. All values are the average of 2 hydrolyses, with no added scavengers. The value for D-Trp was not determined. The peak for Cha overlapped the $NH_3$ peak in the amino acid analysis, so Cha was not determined.

## Example 5

### D-Seryl-glycyl-L-cysteinyl-L-lysyl-L-alanyl-L-phenylalanyl-L-phenylalanyl-D-tryptophyl-L-lysyl-L-threonyl-L-phenylalanyl-L-threonyl-L-seryl-D-cysteine.

The protected peptido-resin prepared in Example D (6.029 g., at a substitution level of 0.162 mmole/g. resin) is mixed with anisole (11 ml.) and ethyl mercaptan (11 ml). The mixture is cooled with liquid nitrogen and liquid hydrogen fluoride (150 ml.) is added by distillation. The mixture is then brought to 0°C. and is stirred for 1.5 hours. Removal of hydrogen fluoride by distillation gives a residue to which is added ether at 0°C. The solid is collected, washed with ether, and dried. The peptide is separated from the resin by extracting the solid with 1M acetic acid and 50% acetic acid. The extract is lyophilized in the dark to dryness. A mixture of 0.2M acetic acid (10 ml) and glacial acetic acid (4 ml) is added to the dry material, and the suspension is warmed to effect solution. Upon cooling, a small amount of precipitate separates. This is not removed by filtration. The sample is chromatographed through a Sephadex G-25 Fine column under the following conditions--solvent: degassed 0.2M acetic acid; column size: 7.5 X 150 cm; temperature: 26°C; flow rate: 1644 ml/hour; fraction volume: 24.67 ml.

A plot of absorbance at 280 mμ of each fraction versus fraction number shows a broad peak with a back-side shoulder. UV spectrographic analysis indicates that the fractions represented by the broad peak contain the desired product. Hence, fractions 205-230 (5081-5755 ml, peak 5506 ml) are combined. UV spectrographic analysis of a sample of the combined fractions indicates that 524 mg. of the product is obtained. Free sulfhydryl content: 91.1% of theoretical (by Ellman titration)

## Example 6

D-Seryl-glycyl-L-cysteinyl-L-lysyl-L-alanyl-L-phenylalanyl-L-phenylalanyl-D-tryptophyl-L-lysyl-L-threonyl-L-phenylalanyl-L-threonyl-L-seryl-D-cysteine cyclic (3 -> 14) disulfide.

The linear peptide prepared in Example 5 is air oxidized to the corresponding cyclic peptide by the following procedure:

A sample of the combined fractions obtained in Example 5, theoretically containing about 260 mg. of peptide, are diluted with distilled water to achieve a final solution having a concentration of 50 μg/ml. Sufficient concentrated ammonium hydroxide is added to

X-5201                           -37-

bring the pH to 6.7. The solution is then stirred at room temperature in the dark for 41 hours at which time Ellman titration of an aliquot indicates complete oxidation.

The solution is concentrated in vacuo to a small volume. Glacial acetic acid (to 31 ml) is added and the solution is desalted by chomatography on a Sephadex G-25 Fine Column under the following conditions -- solvent: degassed 50% acetic acid; column size: 5.0 X 215 cm; temperature: 26°C; flow rate: 243 ml/hour; fraction volume: 21.6 ml.

A plot of absorbance at 280 mμ of each fraction versus fraction number shows several large overlapping peaks. Fractions 93-110 (2044-2508 ml.) are combined and lyophilized to dryness in the dark. The resulting solid is dissolved in degassed 50% acetic acid and is rechromatographed on a column of Sephadex G-25 Fine, as before. The fractions eluted from 2034 to 2214 ml were pooled and lyophilized to dryness in the dark.

The resulting solid is dissolved in degassed 0.2M acetic acid (12 ml.) and the solution is chromatographed on a Sephadex G-25 Fine column under the following conditions -- solvent: degassed 0.2M acetic acid; column size: 2.5 X 215 cm; temperature: 26°C; flow rate: 119 ml/hour; fraction volume: 12.5 ml.

A plot of absorbance at 280 mμ of each fraction versus fraction number shows a single peak. UV spectrographic analysis indicates that the fractions represented by the main part of this peak comprise the desired product.  Fractions 82-89 (1011-1111 ml;
peak, 1034 ml) are combined and lyophilized in the
dark to give the title peptide.  UV spectrographic
analysis of the combined fractions before lyophilization
indicated that 24.4 mg. of the product is obtained.
Recovery, 9% (from the linear form).
Amino acid analysis:
Gly, 0.98; Cys, 1.50; Lys, 2.0; Ala, 1.03; Phe, 2.89;
Thr, 1.84; Ser, 1.62.

The above results are expressed as ratios to
Lys/2.  All values are the average of 2 hydrolyses,
with no added scavengers.  The value for D-Trp was
not determined.

### Example 7

D-Seryl-glycyl-L-cysteinyl-L-lysyl-L-alanyl-L-
phenylalanyl-L-phenylalanyl-D-tryptophyl-L-lysyl-L-
threonyl-D-phenylalanyl-L-threonyl-L-seryl-D-cysteine.

The protected peptido-resin prepared in
Example E (3.511 g., at a substitution level of 0.158
mmole/g. resin) is mixed with anisole (6.4 ml.) and
ethyl mercaptan (6.4 ml).  The mixture is cooled with
liquid nitrogen and liquid hydrogen fluoride (73 ml.)
is added by distillation.  The mixture is then brought
to 0°C. and is stirred for 1.5 hours.  Removal of
hydrogen fluoride by distillation gives a residue to
which is added ether at 0°C.  The solid is collected,
washed with ether, and dried.  The peptide is separated
from the resin by extracting the solid with 1M acetic
acid and 50% acetic acid.  The extract is lyophilized
in the dark to dryness.  A mixture of 0.2M acetic acid (10 ml) and glacial acetic acid (4 ml) is added to the dry material, and the suspension is warmed to effect solution. Upon cooling, a small amount of precipitate separates. Addition of 5 ml. of glacial acetic acid followed by warming effected solution. The sample is chromatographed through a Sephadex G-25 Fine column under the following conditions--solvent: degassed 0.2M acetic acid; column size: 7.5 X 150 cm; temperature: 26°C; flow rate: 1602 ml/hour; fraction volume: 24.4 ml.

A plot of absorbance at 280 mμ of each fraction versus fraction number shows a broad peak. UV spectrographic analysis indicates that the fractions represented by the broad peak contain the desired product. Hence, fractions 217-242 (5227-5870 ml, peak 5581 ml) are combined. UV spectrographic analysis of a sample of the combined fractions indicates that 388.8 mg. of the product is obtained. Free sulfhydryl content: 86% of theoretical (by Ellman titration)

## Example 8

D-Seryl-glycyl-L-cysteinyl-L-lysyl-L-alanyl-L-phenylalanyl-L-phenylalanyl-D-tryptophyl-L-lysyl-L-threonyl-D-phenylalanyl-L-threonyl-L-seryl-D-cysteine cyclic (3 -> 14) disulfide.

The linear peptide prepared in Example 7 is air oxidized to the corresponding cyclic peptide by the following procedure:

The combined fractions obtained in Example 7 (643 ml, theoretically containing 388.8 mg. of peptide) are diluted with 7137 ml of distilled water to achieve a final solution having a concentration of 50 µg/ml.
Sufficient concentrated ammonium hydroxide is added to
bring the pH to 6.7. The solution is then stirred at
room temperature in the dark for about 40 hours at
which time Ellman titration of an aliquot indicates
complete oxidation.

The solution is concentrated _in vacuo_ to a
volume of about 35 ml. Glacial acetic acid (35 ml)
is added and the solution is desalted by chomatography
on a Sephadex G-25 Fine Column under the following
conditions -- solvent: degassed 50% acetic acid;
column size: 5.0 X 215 cm; temperature: 26°C; flow
rate: 243 ml/hour; fraction volume: 22.8 ml.

A plot of absorbance at 280 mµ of each
fraction versus fraction number shows two large peaks.
The first represents the aggregated forms of the
peptide, while the second represents monomeric material.
Fractions 90-125 (2034-2797 ml.) are combined and
lyophilized to dryness in the dark. The resulting
solid is divided into two approximately equal portions
and each is taken up in about 25 ml of 50% acetic
acid and rechromatographed as before in 50% acetic
acid. The corresponding pools from the two reruns
are lyophilized to dryness in the dark.

The resulting solids are dissolved in
degassed 0.2M acetic acid (15 ml.) and the solution is
chromatographed on a Sephadex G-25 Fine column under
the following conditions -- solvent: degassed 0.2M
acetic acid; column size: 5.0 X 150 cm; temperature:
26°C; flow rate: 450 ml/hour; fraction volume: 15.75 ml.

A plot of absorbance at 280 mμ of each fraction versus fraction number shows a single peak. UV spectrographic analysis indicates that the fractions represented by the main part of this peak comprise the desired product. Fractions 171-181 (2688-2864 ml; peak, 2757 ml) are combined and lyophilized in the dark to give the title peptide. UV spectrographic analysis of the combined fractions before lyophilization indicated that 86 mg. of the product is obtained. Recovery, 22.1% (from the linear form).

Amino acid analysis:

Gly, 0.97; Cys, 1.51; Lys, 2.04; Ala, 1.03; D- and L-Phe, 2.85; Thr, 1.83; Ser, 1.69.

The above results are expressed as ratios to Ala + Gly/2. All values are the average of 2 hydrolyses, with no added scavengers. The value for D-Trp was not determined.

## Example 9

D-Seryl-glycyl-L-cysteinyl-L-lysyl-L-alanyl-L-phenylalanyl-L-phenylalanyl-D-tryptophyl-L-lysyl-L-threonyl-L-cyclohexylalanyl-L-threonyl-L-seryl-D-cysteine.

The protected peptido-resin prepared in Example F (5.64 g., at a substitution level of 0.169 mmole/g. resin) is mixed with anisole (10.3 ml.) and ethyl mercaptan (10.3 ml). The mixture is cooled with liquid nitrogen and liquid hydrogen fluoride (72 ml.) is added by distillation. The mixture is then brought to 0°C. and is stirred for two hours. Removal of hydrogen fluoride by distillation gives a residue to which is added ether at 0°C.  The solid is collected, washed with ether, and dried.  The peptide is separated from the resin by extracting the solid with 1M acetic acid and 50% acetic acid.  The extract is lyophilized in the dark to 25 ml.  A solution of 0.2M acetic acid (275 ml) is added to the solution of peptide, and the suspension is warmed to effect solution.  The filtrate is chromatographed through a Sephadex G-25 Fine column under the following conditions--solvent: degassed 0.2M acetic acid; column size: 10 X 210 cm; temperature: 26°C; flow rate: 2626 ml/hour; fraction volume: 65.6 ml.  A 11,869-ml sample is collected in large flasks and discarded before individual fractions are collected.

A plot of absorbance at 280 mµ of each fraction versus fraction number shows a broad peak with a back-side shoulder.  UV spectrographic analysis indicates that the fractions represented by the broad peak contain the desired product.  Hence, fractions 18-42 (12991-14626 ml, peak 13854 ml) are combined.  UV spectrographic analysis of a sample of the combined fractions indicates that 742.5 mg. of the product is obtained.  Recovery: 48.2%; free sulfhydryl content: 93.5% of theoretical (by Ellman titration)

## Example 10

D-Seryl-glycyl-L-cysteinyl-L-lysyl-L-alanyl-L-phenylalanyl-L-phenylalanyl-D-tryptophyl-L-lysyl-L-threonyl-L-cyclohexylalanyl-L-threonyl-L-seryl-D-cysteine cyclic (3 -> 14) disulfide.

The linear peptide prepared in Example 9 is air oxidized to the corresponding cyclic peptide by the following procedure:

The combined fractions obtained in Example 9 (1635 ml, theoretically containing 742.5 mg. of peptide) are diluted with 13,215 ml of distilled water to achieve a final solution having a concentration of 50 µg/ml. Sufficient concentrated ammonium hydroxide is added to bring the pH to 6.7. The solution is then stirred at room temperature in the dark for 41 hours at which time Ellman titration of an aliquot indicates complete oxidation.

The solution is concentrated _in vacuo_ to a small volume and 50% acetic acid is added to a total volume of about 250 ml. The solution is desalted by chomatography on a Sephadex G-25 Fine Column under the following conditions -- solvent: degassed 50% acetic acid; column size: 7.5 X 211 cm; temperature: 26°C; flow rate: 1200 ml/hour; fraction volume: 20.2 ml.

A plot of absorbance at 280 mµ of each fraction versus fraction number shows a low, broad peak. Fractions 35-135 (3577-5619 ml.) are combined and lyophilized to dryness in the dark. The resulting solid is dissolved in degassed 0.2M acetic acid (25 ml.) and the solution is chromatographed on a Sephadex G-25 Fine column under the following conditions -- solvent: degassed 0.2M acetic acid; column size: 5.0 X 215 cm; temperature: 26°C; flow rate: 860 ml/hour; fraction volume: 15.8 ml. A 2120 ml sample is collected in a large flask and discarded before individual fractions are collected.

A plot of absorbance at 280 mµ of each fraction versus fraction number shows a single peak. UV spectrographic analysis indicates that the fractions represented by the main part of this peak comprise the desired product. Fractions 97-115 (3634-3935 ml; peak, 3720 ml) are combined and lyophilized in the dark to give the title peptide. UV spectrographic analysis of the combined fractions before lyophilization indicated that 164.1 mg. of the product is obtained. Recovery, 22.1% (from the linear form).

Amino acid analysis:

Gly, 0.89; Cys, 1.75; Lys, 2,0; Ala, 0.91; Phe, 1.87; Thr, 1.81; Ser, 1.65.

The above results are expressed as ratios to Lys/2. All values are the average of 2 hydrolyses, with no added scavengers. The value for D-Trp was not determined. The peak for Cha overlapped the $NH_3$ peak in the amino acid analysis, so Cha was not determined.

The effects of the tetradecapeptides of Formula III on the inhibition of growth hormone, insulin, and glucagon can be elicited and demonstrated in the following test procedures:

A.  Growth Hormone Inhibition In Rats:

This test is a modification of the method of P. Brazeau et al. Endocrinology, 94, 184 (1974). Male rats (weighing 100-110 g.) are divided into three groups of eight rats each. Each rat is administered sodium pentobarbital at a dose of 4 mg/rat (I.P.) to stimulate growth hormone (GH) secretion. Simultaneously, one group of rats receives the test compound (S.C.); the second group receives somatostatin (S.C.); and the third group (control) receives saline (S.C.). Twenty- minutes later, the animals are decapitated and blood samples are collected. The serum concentration of growth hormone (GH) is determined by radioimmunoassay. The mean GH concentration ($\pm$ standard error of the mean) is calculated for each group. The percent inhibition of GH release (as compared to saline controls) is then calculated for the test compound and for somatostatin. When tested as above-described, the peptides of Examples 2, 4, 6, 8 and 10, representative of the peptides of Formula III, gave the results set forth below in Table I:

## TABLE I

### Growth Hormone Inhibition

(A)  D-Ala$^1$, D-Ala$^5$, D-Trp$^8$, D-Cys$^{14}$-Somatostatin (Example 2)

| Exp. | Dose (µg/kg) | GH Serum Conc. (ng/ml)* Saline | Somatostatin | Compound | % Inhibition Somatostatin | Compound |
|---|---|---|---|---|---|---|
| 1 | 50 | -- | 65.6 ± 18.6 | 2.0 ± 0.3 | 52.5 | 98.6 |
| | 2 | -- | 84.1 ± 18.5 | 7.1 ± 3.8 | 39.1 | 94.9 |
| | -- | 138.2 ± 33.5 | -- | -- | -- | -- |
| 2 | 50 | -- | 34.3 ± 20.1 | -- | 57.9 | -- |
| | 2.5 | -- | -- | 14.8 ± 10.9 | -- | 81.8 |
| | 2 | -- | 37.5 ± 11.9 | -- | 54 | -- |
| | 0.25 | -- | -- | 51.0 ± 14.1 | -- | 37.4 |
| | | 8.15 ± 34.5 | -- | -- | -- | -- |
| 3 | 50 | -- | 20.1 ± 13.5 | -- | 86.7 | -- |
| | 25 | -- | -- | 2.8 ± 0.2 | -- | 98.2 |
| | 2 | -- | 27.4 ± 11.9 | -- | 81.9 | -- |
| | 1 | -- | -- | 30.6 ± 16.0 | -- | 79.8 |
| | -- | 151.5 ± 40.9 | -- | | | |

TABLE I (cont.)

(B) D-Ala$^1$, D-Ala$^5$, D-Trp$^8$, L-Cha$^{11}$, D-Cys$^{14}$-Somatostatin (Example 4)

| Exp. | Dose (µg/kg) | GH Serum Conc. (ng/ml)* | | | % Inhibition | |
| --- | --- | --- | --- | --- | --- | --- |
| | | Saline | Somatostatin | Compound | Somatostatin | Compound |
| 1 | 50 | -- | 10.3 ± 1.6 | 8.4 ± 3.4 | 91 | 92 |
| | 2 | -- | 80.6 ± 28.1 | 5.5 ± 0.7 | 27 | 95 |
| | | 109.6 ± 40.2 | -- | -- | -- | -- |

(C) D-Ser$^1$, Ala$^5$, D-Trp$^8$, D-Cys$^{14}$-Somatostatin (Example 6)

| Exp. | Dose (µg/kg) | GH Serum Conc. (ng/ml)* | | | % Inhibition | |
| --- | --- | --- | --- | --- | --- | --- |
| | | Saline | Somatostatin | Compound | Somatostatin | Compound |
| 1 | 50 | -- | 5.5 ± 1.4 | 4.0 ± 0.5 | 93 | 95 |
| | 2 | -- | 32.0 ± 9.9 | 15.5 ± 5.4 | 56 | 79 |
| | -- | 73.2 ± 36.7 | -- | -- | -- | -- |

## TABLE I (cont.)

(D) D-Ser[1], Ala[5], D-Trp[8], D-Phe[11], D-Cys[14]-Somatostatin (Example 8)

| Exp. | Dose (µg/kg) | GH Serum Conc. (ng/ml)* | | | % Inhibition | |
|------|-------------|--------|--------------|----------|--------------|----------|
|      |             | Saline | Somatostatin | Compound | Somatostatin | Compound |
| 1    | 50          | --     | 24.7 ± 17.8  | 40.0 ± 13.4 | 64        | 39       |
|      | 2           | --     | 76.8 ± 21.0  | 79.6 ± 36.2 | 0         | 0        |
|      | --          | 66.0 ± 32.9 | --      | --       |              |          |

(E) D-Ser[1], Ala[5], D-Trp[8], Cha[11], D-Cys[14]-Somatostatin (Example 10)

| Exp. | Dose (µg/kg) | GH Serum Conc. (ng/ml)* | | | % Inhibition | |
|------|-------------|--------|--------------|----------|--------------|----------|
|      |             | Saline | Somatostatin | Compound | Somatostatin | Compound |
| 1    | 50          | --     | 2.0 ± .1     | 2.2 ± 0.1 | 98         | 98       |
|      | 2           | --     | 74.6 ± 27.3  | 68.8 ± 28.2 | 28       | 34       |
|      | --          | 103.5 ± 36.3 | --     | --       |              |          |

* Mean ± SEM

### B. Insulin and Glucagon Inhibition In Dogs:

A normal dog is fasted overnight. An intravenous (I.V.) infusion of the test compound (dissolved in saline) is begun. Thirty minutes thereafter an additional infusion of L-alanine (dissolved in saline) is begun and is continued for a total of 15 minutes so that a total dose of about 1 mmole of L-alanine per kg. body weight is given. Infusion of the test compound is continued for an additional 15 minute period.

Blood samples are taken periodically before (at -20, -10 and -1 minutes) and after (5, 10, 15, 30, 35, 40, 45, 50, 60, 90, 120 and 150 minutes) the start of the infusion of the test compound. The serum insulin and serum glucagon concentrations in the blood are determined by radioimmunoassay. A plot is made of the glucagon and insulin concentrations versus time. The plot obtained from the test compound is compared to plots obtained from somatostatin and saline (controls) in similar experiments.

Infusion of L-alanine (in the absence of somatostatin or active test compound) causes an abrupt increase in serum insulin and glucagon concentrations. The concentrations return to basal levels after the L-alanine infusion is terminated. Infusion of somatostatin (alone) causes a decrease in basal serum concentrations of insulin and glucagon. In the presence of L-alanine, somatostatin inhibits the increase in insulin and glucagon councentrations induced by the L-alanine.

X-5201                          -50-


        When tested according to the procedure
above-described the peptides of Examples 2, 4, 6, 8
and 10 gave the following results:

|          | Infusion |        |
|----------|----------|--------|
| Peptide  | Dose (µg/kg/min) | Result |
| Example 2 | 0.098<br>0.181<br>0.291 | No inhibition of glucagon and insulin |
| Example 4 | 0.05 | No inhibition of glucagon and insulin |
| Example 6 | 0.05 | Significant inhibition of glucagon and insulin |
| Example 8 | 0.20 | No inhibition of glucagon and insulin |
| Example 10 | 0.06 | Partial inhibition of glucagon and insulatum. |

X-5201-1 -51-

CLAIMS

1. A cyclic tetradecapeptide of the formula

X-Cys-Lys-Y-Phe-Phe-D-Trp
|                 |
HO-D-Cys—Ser-Thr-Z-Thr—Lys    III

wherein:

    X is H-Ala-Gly, H-Ala-D-Ala, H-D-Ala-Gly, H-D-Val-Gly, H-Ala-D-Val, H-Ala-D-Ser, or H-D-Ser-Gly;

    Y is Ala or D-Ala; and

    Z is Phe, D-Phe, or Cha;

or a non-toxic, pharmaceutically acceptable acid addition salt thereof.

2. 
H-D-Ala-Gly-Cys-Lys-D-Ala-Phe-Phe-D-Trp
|           |
HO-D-Cys—Ser-Thr—Phe—Thr—Lys   ;

H-D-Ala-Gly-Cys-Lys-D—Ala-Phe-Phe-D-Trp
|           |
HO-D-Cys—Ser—Thr—Cha—Thr—Lys  ;

H-D-Ser-Gly-Cys-Lys-Ala-Phe-Phe-D-Trp
|           |
HO-D-Cys—Ser-Thr-Phe-Thr—Lys  ;

H-D-Ser-Gly-Cys-Lys-Ala-Phe-Phe-D-Trp
|           |
HO-D-Cys-Ser-Thr-D-Phe-Thr-Lys  or

H-D-Ser-Gly-Cys-Lys-Ala-Phe-Phe-D-Trp
|           |
HO-D-Cys-Ser—Thr-Cha-Thr—Lys

or a non-toxic, pharmaceutically acceptable acid addition salt thereof.

3.  A linear tetradecapeptide of the formula:

X-Cys-Lys-Y-Phe-Phe-D-Trp
                        |              IV
HO-D-Cys-Ser-Thr-Z-Thr-Lys

wherein:

X is H-Ala-Gly, H-Ala-D-Ala, H-D-Ala-Gly,
H-D-Val-Gly, H-Ala-D-Val, H-Ala-D-Ser, or
H-D-Ser-Gly;

Y is Ala or D-Ala; and

Z is Phe, D-Phe, or Cha;

or a non-toxic, pharmaceutically acceptable acid
addition salt thereof.

4.  H-D-Ala -Gly-Cys-Lys-D-Ala-Phe-Phe-D-Trp
                                          |       ;
      HO-D-Cys-Ser-Thr-Phe-Thr-Lys

    H-D-Ala -Gly-Cys-Lys-D-Ala-Phe-Phe-D-Trp
                                          |       ;
      HO-D-Cys-Ser-Thr-Cha-Thr-Lys

    H-D-Ser-Gly-Cys-Lys-Ala-Phe-Phe-D-Trp
                                        |       ;
      HO-D-Cys-Ser-Thr-Phe-Thr-Lys

    H-D-Ser-Gly-Cys-Lys-Ala-Phe-Phe-D-Trp
                                        |       or
      HO-D-Cys-Ser-Thr-D-Phe-Thr-Lys

    H-D-Ser-Gly-Cys-Lys-Ala-Phe-Phe-D-Trp
                                        |
      HO-D-Cys-Ser-Thr-Cha-Thr-Lys

or a non-toxic pharmaceutically acceptable acid addition salt thereof.

5.  A process for the preparation of a cyclic
tetradecapeptide of the formula

X-Cys-Lys-Y-Phe-Phe-D-Trp
          |                    |         III
HO-D-Cys—Ser-Thr—Z-Thr-Lys

wherein:

  X is H-Ala-Gly, H-Ala-D-Ala, H-D-Ala-Gly,
   H-D-Val-Gly, H-Ala-D-Val, H-Ala-D-Ser,
   or H-D-Ser-Gly;

  Y is Ala or D-Ala; and

  Z is Phe, D-Phe, or Cha;

or a non-toxic, pharmaceutically acceptable acid addition salt thereof, which comprises reacting a linear peptide of the formula

$$X\text{-}Cys\text{-}Lys\text{-}Y\text{-}Phe\text{-}Phe\text{-}D\text{-}Trp$$
$$HO\text{-}D\text{-}Cys\text{-}Ser\text{-}Thr\text{-}Z\text{-}Thr\text{-}Lys \qquad IV$$

wherein the various symbols are defined as above, with an oxidizing agent.

  6.  A process of claim 5 wherein the oxidizing agent is air.

  7.  A pharmaceutical formulation which comprises as active ingredient a compound of Formula III as defined in claim 1, with a pharmaceutically acceptable carrier.

  8.  A compound of claim 1 or 2 for use as a pharmaceutical.

  9.  A compound of claim 1 or 2 for use as a growth hormone inhibiting agent.